# EUROPÄISCHE PATENTANMELDUNG

(11) **EP 2 505 505 A2**
(43) Veröffentlichungstag der Anmeldung: **03.10.2012**
(21) Anmeldenummer: 12160405.2
(22) Anmeldetag: 20.03.2012
(51) Int. Cl.: B65B 55/08, B65B 65/00

(54) **Verfahren und Vorrichtung zum Sterilisieren von Verpackungsmitteln**

(30) Priorität: 28.03.2011 DE 102011015344
(71) Anmelder: Krones AG, 93073 Neutraubling (DE)
(72) Erfinder: Humele, Heinz, 93107 Thalmassing (DE); Kraus, Andreas, 93049 Regensburg (DE); Laumer, Roland, 93047 Regensburg (DE); Folz, Cornelia, 13353 Berlin (DE); Forsthövel, Jochen, 93059 Regensburg (DE); Engelhard, Patrick, 84094 Elsendorf (DE); Sonnauer, Andreas, 93086 Wörth (DE)
(74) Vertreter: Bittner, Bernhard

(57) **Zusammenfassung**

Ein Verfahren zum Sterilisieren von Verpackungsmitteln (10) und insbesondere von Kunststoffvorformlingen (10), wobei die Verpackungsmittel (10) mit einer Transporteinrichtung (2) transportiert werden und wobei wenigstens ein Bereich des Verpackungsmittels (10) mit einer Beaufschlagungseinrichtung (4) mit einem fließfähigen Medium beaufschlagt wird. Erfindungsgemäß wird das auf dem Bereich aufgebrachte Medium einer Bestrahlung mit elektromagnetischer Strahlung ausgesetzt, um Singulett - Sauerstoff zu erzeugen wobei dieser Singulett - Sauerstoff zum Sterilisieren des Verpackungsmittels (10) dient.

## Beschreibung

Die vorliegende Erfindung bezieht sich auf ein Verfahren und eine Vorrichtung zum Sterilisieren von Verpackungsmitteln. Im Bereich der getränkeherstellenden Industrie ist es seit langem bekannt, dass Verpackungsmittel, wie beispielsweise Glas- oder Kunststoffbehältnisse, Verpackungsfolien, Behältnisverschlüsse und dergleichen sterilisiert werden, bevor sie beispielsweise mit einem Getränk befüllt werden. Hierzu sind aus dem Stand der Technik die unterschiedlichsten Sterilisationsverfahren bekannt. So ist es beispielsweise bekannt, die jeweiligen Verpackungsmitteln mit Peressigsaure oder H₂O₂ zur Sterilisation zu beaufschlagen. Auch ist es bekannt, die Verpackungsmittel mit ultraviolettem Licht oder Röntgenstrahlen und dergleichen zu beaufschlagen. Im Falle der sogenannten Nassentkeimung bei der Peressigsäure verwendet wird oder der Trockensterilisation mit dem Einsatz von H₂O₂ besteht der Nachteil, dass es sich hierbei um aggressive Medien handelt, die relativ schwierig zu behandeln sind und daher eine aufwendige Maschinentechnik erfordern. Daneben ist der Energieaufwand relativ hoch und der Trinkwasserkreislauf wird mit Chemikalien belastet.

Aus der DE 60 2004 011 056 T2 sind ein Verfahren und eine Vorrichtung zur Bestrahlung mittels Elektronenstrahlen bekannt. Dabei wird der Behälter einer Elektronenstrahlung unterworfen und innerhalb des Behälters Ozon durch die Umwandlung von atmosphärischem Sauerstoff erzeugt. Dieses Ozon wird für eine ausreichende Zeitdauer in dem geschlossenem Behälter gehalten um diesen zu sterilisieren.

Aus der DE 697 15 399 T2 sind ebenfalls ein Verfahren und eine Vorrichtung zum Sterilisieren von Nahrungsmittelbehältern bekannt. Dabei wird der Innenraum des Behälters mit ultraviolettem Licht kurz nach dem Aufbringen einer Wasserstoffperoxyd (H₂O₂) enthaltenden Lösung bestrahlt.

Die DE 697 35 141 T2 beschreibt ebenfalls ein Verfahren zum Sterilisieren eines Behälters, wobei der Behälter einer Elektronenbestrahlung unterworfen wird und Ozon innerhalb des Behälters durch die Umwandlung von atmosphärischem Sauerstoff erzeugt wird.

Diese genannten Verfahren, welche Elektronenstrahlung oder UV-Strahlung einsetzen, haben jedoch wiederum den Nachteil, dass die besagten Strahlungen, da sie auch für den Benutzer schädlich sein können, abzuschirmen sind. Auch diese entsprechenden Abschirmungsmechanismen sind dabei vergleichsweise aufwendig.

Der vorliegenden Erfindung liegt daher die Aufgabe zugrunde, ein Verfahren und eine Vorrichtung zum Sterilisieren von Verpackungsmitteln zur Verfügung zu stellen, welches einerseits ohne aggressive Substanzen auskommt und andererseits auch ohne den Einsatz für Menschen schädlicher (z.B. ionisierender) Strahlung.

Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Bei einem Verfahren zum Sterilisieren von Verpackungsmitteln und insbesondere von Kunststoffvorformlingen werden die Verpackungsmittel mit einer Transporteinrichtung transportiert und wenigstens ein Bereich des Verpackungsmittels mit einer Beaufschlagungseinrichtung mit einem fließfähigen Medium beaufschlagt. Bevorzugt handelt es sich bei dem fließfähigen Medium um einen in Lösung (bevorzugt in Wasser) vorliegenden Photosensibilisator.

Erfindungsgemäß wird das auf dem Bereich aufgebrachte Medium einer Bestrahlung mit elektromagnetischer Strahlung ausgesetzt um Singulett Sauerstoff zu aktivieren bzw. zu erzeugen, wobei dieser Singulett-Sauerstoff zum Sterilisieren des Verpackungsmittels dient. Dabei kann das fließfähige Medium Sauerstoff enthalten, bevorzugt wird jedoch der Sauerstoff aus der Umgebung genutzt. Vorteilhaft handelt es sich bei dem fließfähigen Medium um eine Flüssigkeit.

Es wird daher im Vergleich zum Stand der Technik eine alternative Vorgehensweise vorgeschlagen, wobei während des eigentlichen Sterilisationsvorgangs sogenannter Singulett - Sauerstoff erzeugt wird. Bevorzugt wird durch die Beaufschlagung mit der elektromagnetischen Strahlung ein photodynamischer Prozess ausgelöst. Ein wesentlicher Mechanismus in der Photodynamik ist die Absorption von Licht in Farbstoffmolekülen, welche auch als Photosensibilisatoren bezeichnet werden sowie die daraufhin ablaufenden möglichen Prozesse. So kann das absorbierte Licht in diesem Photosensibilisator in Hitze umgewandelt oder wieder als Fluoresenzlicht abgegeben werden. Für den dritten und für die Desinfektion bzw. Sterilisation entscheidenden Prozess kann ein Teil der absorbierten Lichtenergie auf den umliegenden Sauerstoff übertragen werden, wodurch sich eine äußerst reaktive Sauerstoffspezies ausbildet. Der so generierte Singulett Sauerstoff kann anschließend die Zellwände von Bakterien und anderen Mikroorganismen irreparabel schädigen und damit abtöten. Welcher der drei genannten Prozesse dominiert, kann durch die geeignete Wahl des Photosensibilisators und die Lichtdosimetrie gesteuert bzw. beeinflußt werden.

Es wird vorgeschlagen, dieses aus dem Stand der Technik an sich bekannte Verfahren zur Sterilisation von Verpackungsmitteln und insbesondere zur Sterilisation von Kunststoffvorformlingen oder deren Verschlüssen und von Maschinen- und Maschinenteile zu verwenden. Ein Vorteil dieses Verfahrens, bei dem unter anderem auch Farbstoffe bzw. auch Vitamine zum Einsatz kommen können, besteht darin, dass Photosensibilisatoren nach der Applikation auf dem zu sterilisierenden Objekt verbleiben. Es wäre jedoch auch möglich, die Photosensibilisatoren anschließend abzutragen, beispielsweise durch einen Spülvorgang mit Sterilwasser oder Sterilluft. Damit handelt es sich vorteilhaft bei dem fließfähigen Medium um einen sogenannten Photosensibilisator. Ein Vorteil des Verfahrens besteht darin, dass derartige Photosensibilisatoren in der Regel lebensmitteltauglich sind und beispielsweise Vitamine als Photosensibilisatoren in Frage kommen. Auf diese Weise ist es auch möglich, Spülvorgänge zu reduzieren oder gar abzuschaffen. Es ist weiterhin möglich, dass die oben erwähnte Aktivierung der photodynamischen Prozesse erst kurz vor der Verarbeitung des zu sterilisierenden Körpers stattfindet bzw. während oder nach der Verarbeitung. Sofern der Photosensibilisator photostabil ist, also nach Bestrahlung nicht zerfällt, können die oben genannten Effekte mehrmals angestoßen werden

Schließlich kann durch die erfindungsgemäße Vorgehensweise die Maschinentechnik vereinfacht werden und ggfs. auf den Einsatz von Chemikalien verzichtet werden. So kann beispielsweise der Sterilisationsprozess durch die Bestrahlung erst unmittelbar vor einem eigentlichen Befüllprozess von Behältern angestoßen werden, wodurch eine verfahrens- wie auch maschinentechnisch aufwendige Sterilzone verkleinerbar ist. Ein weiterer vorteilhafter Nutzen einer photostabilen Farbstofflösung ist die wiederholbare Entkeimung von Maschinen bzw. Maschinenteilen. Diese ist vor allem für die Aufrechterhaltung der sterilen Zone notwendig.

Bei der Beaufschlagung der Verpackungsmittel handelt es sich insbesondere um eine Benetzung der jeweiligen Oberflächenbereiche, wobei jedoch auch ein Eintauchen des Verpackungsmittels in ein Bad des fließfähigen Mediums möglich ist. Die Tauchbadbehandlung kann ggf. durch Ultraschalleinsatz unterstützt werden. Daneben ist es auch möglich, dass die besagten Bereiche mit dem fließfähigen Medium besprüht werden. Bevorzugt wird die gesamte Innenoberfläche eines zu sterilisierenden Verpackungsmittels benetzt bzw. das fließfähige Medium auf der gesamten Innenoberfläche verteilt. Vorteilhaft bleibt diese Benetzung während des gesamten folgenden Prozesses und insbesondere auch während der Bestrahlung erhalten. Vorteilhaft bleibt die Benetzung während des gesamten Prozesses auch im Wesentlichen konstant. Bevorzugt wird zu diesem Zweck das fließfähige Medium in der Weise modifiziert, dass es besser an den Flächen des Verpackungsmittels bzw. an den Mikroorganismen haften bleibt. Daher kann es vorteilhaft sein, die Fließfähigkeit und Benetzungsfähigkeit des fließfähigen Mediums anzupassen oder zu verändern.

Vorteilhaft handelt es sich bei den besagten Bereichen des Verpackungsmittels wenigstens abschnittsweise um eine Innenwandung desselben. Vorteilhaft werden diejenigen Bereiche des Verpackungsmittels bzw. Kunststoffvorformlings mit dem fließfähigen Medium beaufschlagt, welche in einem späteren verschlossenen Zustand des Verpackungsmittels mit der in diesem Verpackungsmittel befindlichen Substanz insbesondere dem in diesem Verpackungsmittel befindlichen Getränk in Berührung kommen.

Bei einem weiteren vorteilhaften Verfahren wird das Verpackungsmittel während des Transports mit dem fließfähigen Medium beaufschlagt und/oder der Singulett - Sauerstoff während des Transports des Verpackungsmittels erzeugt bzw. aktiviert.

Vorteilhaft wird dabei der besagte Bereich des Verpackungsmittels zunächst mit dem fließfähigen Medium beaufschlagt und anschließend erst nach einer bestimmten Zeit (Einwirkzeit) der Singulett Sauerstoff durch die Bestrahlung (insb. elektromagnetische Bestrahlung und besonders bevorzugt Licht im sichtbaren Wellenlängenbereich) aktiviert. Bei einem weiteren vorteilhaften Verfahren verbleibt das Medium auch noch nach der Aktivierung bzw. Bestrahlung einen vorgegebenen Zeitraum auf dem Bereich des Verpackungsmittels, um so eine effiziente Abtötung von Bakterien und Keimen zu erreichen. Bevorzugt wird eine Reduktionsrate der Mikroorganismen in einem Bereich von 5 log - Stufen (also um den Faktor 10000) erreicht, wobei dies insbesondere für diejenigen Bereiche des Verpackungsmittels gilt, welche später mit dem Produkt wie z.B. einem Getränk in Berührung kommen.

Im Rahmen der technischen Umsetzung wäre es möglich, das fließfähige Medium, d. h. einen Photosensibilisator, in das Verpackungsmittel, wie beispielsweise eine Flasche, den Kunststoffvorformling, eine Dose oder auch andere insbesondere rotationssymmetrische druckstabile und nicht druckstabile Hohlkörper einzusprühen. Bei dieser Vorgehensweise kann das aus dem Stand der Technik bekannte Prinzip von Rinsern verwendet werden. Über ein Ein- oder Mehrkanalsystem kann der Farbstoff (der Photosensibilisator) in das Behältnis gesprüht und verteilt werden.. Dabei wäre es möglich, dass das Behältnis über Kopf oder hängend gehalten wird. Der innen und/oder auch außen benetzte Körper bzw. das Verpackungsmittel wird anschließend mit Strahlung und insbesondere mit Licht bestimmter Wellenlänge bestrahlt und somit sterilisiert. Anschließend ist es möglich, dass noch ein Spülvorgang durchgeführt wird. Bei der Bestrahlungseinrichtung kann es sich dabei auch um eine laserbasierte Bestrahlungseinrichtung handeln. Dabei können Laser mit fester oder auch durchstimmbarer Wellenlänge eingesetzt werden. Daneben können auch Blitzlampen, hochenergetische Lampen, Hochdrucklampen, LED's, Kombinationen hieraus und dergleichen Anwendung finden.

Wie oben, enthält das fließfähige Medium vorteilhaft einen Farbstoff. Insbesondere handelt es sich bei dem Medium um einen Farbstoff bzw. eine Farbstofflösung. Daneben wäre es jedoch auch möglich, das fließfähige Medium auf Verpackungsmittel wie Verschlüsse, Versiegelungen, Schüttgüter, Granulate oder Folien, mittels eines Tauchbades aufzubringen und anschließend die so beaufschlagten Verpackungsmittel zu bestrahlen.

Vorzugsweise handelt es sich bei dem fließfähigen Medium um einen Stoff und insbesondere einen Farbstoff, welcher in der Lage ist, den Photodynamischen Effekt hervorzurufen. Bevorzugt enthält der Farbstoff lumineszierende oder reflektierende Partikel, die das zum Aktivieren bzw. Anregen erforderliche Licht auch in schwer zugängliche Bereiche mit beispielsweise hinterschnittenen Kanten projizieren. Die Reflektionspartikel sorgen dafür, dass die Farbschicht diffus reflektiert. Wenn nun ein Lichtstrahl darauf trifft, wird er in alle freien Raumrichtungen reflektiert. Dies ermöglicht es vorteilhafter Weise auch verdeckte Seiten einer Maschine oder die innere Schulterfläche einer Flasche zu erreichen. Bei der Lumineszenzmethode (Fluoreszenz oder Phosphoreszenz) werden Moleküle/Partikel, die lumineszieren, dem Farbstoff zugegeben. Werden diese angestrahlt, fangen sie selbst an ungerichtet zu leuchten und tragen so das Licht in verdeckte Bereiche. Bevorzugt liegt die Anregungswellenlänge der lumineszierenden Moleküle/Partikel niedriger als bei den Farbstoffen, um bei der Emission die Anregungswellenlänge der Farbstoffe zu treffen. Hinzu kommt dass bei phosphoreszierenden Molekülen/Farbstoffen auch durch kurze Anregung die Belichtungsdauer erhöht werden kann (Nachleuchten), und somit noch länger photodynamische Prozesse ablaufen können. Damit können auch verwinkelte Strukturen wirtschaftlich ohne großen Beleuchtungsaufwand erreicht werden.

Bei einem weiteren vorteilhaften Verfahren erfolgt die Beaufschlagung des Verpackungsmittels vor und/oder während und/oder unmittelbar nach einer Erwärmung des Verpackungsmittels. Insbesondere wenn es sich bei dem Verpackungsmittel um einen Kunststoffvorformling handelt, ist es aus dem Stand der Technik bekannt, dass diese mit einer Blasmaschine bzw. Streckblasmaschine zu Kunststoffbehältnissen umgeformt werden. Vor diesem Umformungsprozess werden die Kunststoffvorformlinge üblicherweise mit einer Erwärmungseinrichtung erwärmt, wie beispielsweise einem Infrarot- oder Mikrowellenofen. Es wird dabei vorgeschlagen, dass beispielsweise vor oder während dieses Erwärmungsvorgangs zumindest teilweise zeitgleich die Beaufschlagung des Verpackungsmittels mit dem fließfähigen Medium und ggfs. auch die Aktivierung des Singulett - Sauerstoffs erfolgt. Die Verweilzeit im Ofen kann zur Sterilisierung der vorher mit dem Medium beaufschlagten Vorformlinge genutzt werden. Die Strahlung im Heizprozess wird zur Aktivierung des Sterilisationsprozesses und Aufrechterhaltung bis zum sterilen Befüllen genutzt. Auch wäre es möglich, dass das fließfähige Medium durch die Erwärmungseinrichtung gezielt erwärmt wird (vor oder nach der Bestrahlung), um beispielsweise zu verdampfen. Daneben wäre es auch möglich, dass das fließfähige Medium zwischen einer Heizeinrichtung und einer Streckblasmaschine auf die (Innen-) Wandung des Kunststoffvorformlings ausgebracht wird.

Bei einem weiteren vorteilhaften Verfahren wird das fließfähige Medium dem Verpackungsmittel über eine Zuführleitung und/oder wenigstens eine Düse zugeführt. Aus dem internen Stand der Technik der Anmelderin sind dabei ähnliche Verfahren bekannt, mit denen beispielsweise H₂O₂ oder Peressigsäure oder dergleichen auf die Innenwandung von Kunststoffvorformlingen aufgebracht wird.

Weiterhin wäre es auch möglich, insbesondere bei der Anwendung für Schüttgüter oder Granulate, diese in ein Behältnis wie eine drehbare Trommel einzuführen und dort mit dem fließfähigen Medium zu beaufschlagen. Weitere Verpackungsmittel, wie Verschlüsse oder Versiegelungen können beispielsweise mit einem Fördersystem über Schnecken oder Bahnen in einem geschlossenen System transportiert werden, wobei die Aktivierungsstrahlungsquelle diesem gegenüber abgeschottet ist.

Weiterhin ist es auch möglich, das Verpackungsmittel, insbesondere Kunststoffvorformlinge transportiert werden und an Düsenstöcken vorbeigeführt werden. Durch diese Vorgehensweise wird insbesondere eine Außensterilisation der Kunststoffvorformlinge oder Verpackungsmittel ermöglicht.

Vorteilhaft handelt es ich bei dem fließfähigen Medium um eine positiv oder eine negativ geladene Flüssigkeit. Auf diese Weise kann ein leichteres Anhaften an Mikroorganismen erreicht werden. Allgemein ist vorteilhaft das fließfähige Medium auf die abzutötenden Mikroorganismen abgestimmt. Die Mikroorganismen, die mit dem hier beschriebenen Verfahren abgetötet werden sollen zu sind größtenteils grampositiv d.h. negativ geladen. Für diese Mikroorganismen wird bevorzugt ein positiver Farbstoff verwendet. Einige Mikroorganismen wie z.B. der aspergillus niger können jedoch auch positiv geladen sein, so dass sich hier eine negativ geladene Flüssigkeit bzw. ein negativ geladener Farbstoff anbietet.

Vorteilhaft kann zur Erhöhung der Effektivität und/oder Sicherstellung der Prozessverhältnisse gezielt und unter kontrollierten Bedingungen Sauerstoff dem Prozess hinzugefügt werden. Günstig ist ferner die Nutzung eines Leuchtmittels mit großem Wellenlängenbereich zur Kombinierung der photodynamischen Sterilisation und der Sterilisation durch UV-Licht.

Wenn die Photosensibilisatoren aufgrund ihrer Lebensmitteltauglichkeit (nicht toxischen Wirkung) am/im Verpackungsmittel verbleiben und wenn diese photostabil, d.h. im Prozess immer wieder anstossbar sind, ist es ggf. vorteilhaft, diese Photostabilität nach der Sterilisation durch Überstrahlung und/oder Temperierung gezielt zu zerstören, damit später z.B. im Produkt keine ungewünschte Reaktion mehr stattfindet.

Bei einem weiteren vorteilhaften Verfahren folgen die Beaufschlagung und Sterilisation der Verpackungsmittel in einem kontinuierlichen Prozess, insbesondere auf einer Rundläufermaschine. Dies bedeutet, dass die Verpackungsmittel zumindest abschnittsweise entlang eines kreisförmigen Pfads geführt werden und während dieses Transports auf diesem kreisförmigen Pfad mit dem fließfähigen Medium und besonders bevorzugt auch mit der elektromagnetischen Strahlung beaufschlagt werden.

Vorteilhaft bewegt sich die Beaufschlagungseinrichtung wenigstens abschnittsweise mit dem zu sterilisierenden Verpackungsmittel, wie einem Kunststoffvorformling, mit. Auch wäre es denkbar, dass sich die Bestrahlungseinrichtung wenigstens abschnittsweise mit dem Verpackungsmittel mitbewegt. So könnten beispielsweise auf einem Trägerrad eine Vielzahl von Beaufschlagungseinrichtungen und bevorzugt auch Bestrahlungseinrichtungen angeordnet sein und auf dem gleichen Trägerrad auch Halteeinrichtungen zum Halten der Kunststoffvorformlinge. Dabei kann weiterhin eine Steuerungseinrichtung vorgesehen sein, welche bewirkt, dass bei jeder derartigen Sterilisationsstation der Kunststoffvorformling zunächst mit dem fließfähigen Medium beaufschlagt wird und anschließend nach einer vorgegebenen Zeitspanne die Bestrahlung der beaufschlagten Bereiche einsetzt.

Bei einem weiteren Verfahren wird wenigstens ein Parameter des fließfähigen Mediums, insbesondere vor dessen Applikation auf dem Verpackungsmittel überwacht. So ist es beispielsweise möglich, dass eine Temperatur des fließfähigen Mediums bestimmt wird. Auch wäre es möglich, dass Durchfluss- und Konzentrationsmessungen (insbesondere inline) durchgeführt werden. Auch können Füllhöhenmessungen (z.B. bei Verwendung eines Tauchbads vorgenommen werden.

Daneben wäre es auch möglich, dass ein Verbrauch des fließfähigen Mediums bestimmt wird, insbesondere um so Rückschlüsse auf ausreichend benetzte Verpackungsstücke zu ermöglichen. Auch wäre es möglich, für die Bestrahlung charakteristische Daten zu bestimmen, wie etwa eine Strahlungsenergie oder eine Strahlungsleistung. Auf diese Weise kann eine Bestrahlungsdosis bestimmt werden.

Die vorliegende Erfindung bezieht sich weiterhin auf eine Vorrichtung zum Sterilisieren von Verpackungsmitteln und insbesondere Kunststoffvorformlingen. Diese Vorrichtung weist dabei eine Transporteinrichtung auf, welche das Verpackungsmittel entlang eines vorgegebenen Transportpfades transportiert sowie eine Beaufschlagungseinrichtung, welche wenigstens einen Bereich des Verpackungsmittels mit einem fließfähigen Medium beaufschlagt.

Erfindungsgemäß weist die Vorrichtung weiterhin eine Bestrahlungseinrichtung auf, welche den mit dem fließfähigen Medium beaufschlagten Bereich des Verpackungsmittels einer elektromagnetischen Strahlung zur Erzeugung von Singulett - Sauerstoff aussetzt.

Vorteilhaft weist dabei die Bestrahlungseinrichtung wenigstens eine und bevorzugt eine Vielzahl von Leuchtdioden auf. Es wäre jedoch auch möglich, dass als Lichtquelle (zusätzlich oder alternativ) beispielsweise eine Blitzlampe oder hochenergetische Lampe dient.

Falls es sich bei dem Verpackungsmittel um einen Kunststoffvorformling handelt, wäre es dabei möglich, dass die Strahlungsquelle außerhalb des Kunststoffvorformlings angeordnet ist und diesen von außen her, d. h. durch seine Wandung hindurch zur Aktivierung des Singulett - Sauerstoffs beleuchtet. Es wäre jedoch auch möglich, dass die Lichtquelle in das Innere des Kunststoffvorformlings eingeführt wird. Daneben wäre es auch möglich, dass die Bestrahlungseinrichtung von der Mündung her die Kunststoffvorformlinge bzw. deren Innenwandung bestrahlt. Auch könnten in den Kunststoffvorformling Reflektorelemente eingeführt und von außen beleuchtet werden.

Vorteilhaft transportiert die Transporteinrichtung die Kunststoffvorformlinge entlang einer kreisförmigen Bahn. Daneben oder alternativ wäre es auch möglich, dass Kunststoffvorformlinge auch entlang ihrer Längsrichtung bewegt bzw. transportiert werden. So wäre es möglich, dass die Kunststoffvorformlinge durch eine Heizeinrichtung, wie eine Mikrowellenheizeinrichtung transportiert und währen dieses Vorgangs auch sterilisiert werden. Daneben wäre jedoch auch der Transport der Kunststoffvorformlinge entlang einer zumindest abschnittsweise geradlinigen Bahn möglich.

Bei einer weiteren vorteilhaften Ausführungsform weist die Transporteinrichtung ein Haltemittel zum Halten des Verpackungsmittels auf und in dieses Haltemittel ist bevorzugt auch die Beaufschlagungseinrichtung und/oder die Bestrahlungseinrichtung integriert. Dabei handelt es sich bei dem Haltemittel bevorzugt um ein solches Haltemittel, welches den Kunststoffvorformling an einer Innenwandung kontaktiert. So kann es sich bei dem Haltemittel beispielsweise um einen Dorn handeln, der in eine Mündung des Kunststoffvorformlings eingreift.

Im Bereich dieses Dorns und bevorzugt demjenigen Bereich, der in den Kunststoffvorformling hineinragt, können dabei die besagten Beaufschlagungseinrichtungen beispielsweise in Form von Düsen oder auch Öffnungen angeordnet sein. Auch die Bestrahlungseinrichtung bei der es sich beispielsweise um Leuchtdioden handeln kann, kann in dieses Haltemittel integriert sein.

Damit greift bevorzugt das Haltemittel in eine Mündung des Verpackungsmittels ein. Bevorzugt weist die Vorrichtung eine Vielzahl derartiger Haltemittel auf und diese sind besonders bevorzugt äquidistant zueinander angeordnet. Vorteilhaft ist die Fläche, an der das Haltemittel den Kunststoffvorformling kontaktiert, minimiert. Bei einer weiteren vorteilhaften Ausführungsform sind auch in das Haltemittel Öffnungen integriert, um den Kunsttoffvorformling auch in den berührten Bereichen mit dem fließfähigen Medium zu beaufschlagen.

Bei einer weiteren vorteilhaften Ausführungsform weist die Vorrichtung eine Erwärmungseinrichtung zum Erwärmen des Verpackungsmittels und insbesondere des Kunststoffvorformlings auf. Bei dieser Erwärmungseinrichtung kann es sich in einer besonders bevorzugten Ausführungsform um eine mikrowellenbasierte Erwärmungseinrichtung handeln. Vorteilhaft ist der besagten Vorrichtung in der Transportrichtung der Verpackungsmittel eine Streckblasmaschine nachgeschaltet.

Bei einer weiteren vorteilhaften Ausführungsform wäre es auch möglich, dass der Kunststoffvorformling bezüglich seiner Längsachse und gegenüber der Beaufschlagungseinrichtung gedreht wird. So ist es möglich, die Innenwandung des Kunststoffvorformlings voll umfänglich zu bestrahlen, auch wenn die Beaufschlagungseinrichtung das fließfähige Medium nur in einer (insbesondere schrägen) Richtung abgibt. Es wäre jedoch auch möglich, mehrere Strahlungseinrichtungen zu verwenden, welche den Kunststoffvorformling aus mehreren Richtungen beleuchten. Auf diese Weise können Schattenwürfe, welche in bestimmten Bereichen des Kunststoffvorformlings die Erzeugung des Singulett - Sauerstoffs verhindern, vermieden werden. Zur Optimierung der Ausbeute der Bestrahlung können vorteilhaft Reflektoren, z.B. verspiegelte Flächen eingesetzt werden.

Weiterhin wäre es auch möglich, dass der Kunststoffvorformling schräg entlang einer kreisförmigen Bahn geführt wird. Auf diese Weise ist es möglich, dass bedingt durch die Fliehkraft das fließfähige Medium schneller innerhalb des Kunststoffvorformlings nach unten gelangt und damit die vollständige Innenoberfläche des Kunststoffvorformlings mit dem fließfähigen Medium benetzt wird. Weiterhin wäre es bei dieser Ausführungsform auch möglich, dass eine Bestrahlungseinrichtung vorgesehen ist, der gegenüber sich der Kunststoffvorformling dreht, um so in seiner Umfangsrichtung von allen Seiten bestrahlt zu werden. So könnte beispielsweise auch eine außerhalb des Kunststoffvorformlings angeordnete Lichtleiste angeordnet sein, der gegenüber sich der Kunststoffvorformling dreht. Es wäre jedoch auch möglich, die Lichtquelle ringförmig bzw. ringsegmentförmig auszugestalten und den Kunststoffvorformling durch diese ring(segment)förmige Lichtquelle hindurchzubewegen.

Bei einer weiteren vorteilhaften Ausführungsform weist die Vorrichtung einen Reinraum auf. So ist es möglich, dass die Verpackungsmittel wie Kunststoffvorformlinge während der Beaufschlagung mit dem fließfähigen Medium und bevorzugt auch während der Bestrahlung bereits durch einen derartigen Reinraum oder Sterilraum geführt werden. Auf diese Weise kann die Sterilität des Kunststoffvorformlings verbessert werden. Es wäre jedoch auch möglich, dass sich ein derartiger Reinraum erst unmittelbar an die hier beschriebene Vorrichtung anschließt, sodass die sterilisierten Kunststoffvorformlinge unmittelbar in den besagten Reinraum gelangen. Vorzugsweise gibt die Bestrahlungseinrichtung Licht im sichtbaren Wellenlängenbereich ab.

Da eine gewisse Bestrahlungs- oder Lichtdosis für den Prozess notwendig ist, vorzugsweise mindestens 10 J/cm2, könnte dieser durch Absorption zur Erwärmung des Kunststoffvorformlings führen. Diese Erwärmung kann auch als Energie für den Aufheizvorgang des Kunststoffvorformlings genutzt werden. Es wäre jedoch auch möglich, dass der hier beschriebene Prozess nach der Erwärmung der Kunststoffvorformlinge stattfindet, sodass beispielsweise nach einem Erwärmungsprozess eine temperierte Farbstofflösung in den Kunststoffvorformling eingebracht wird, anschließend einwirken gelassen wird, anschließend der Kunststoffvorformling bestrahlt wird und schließlich in einem Streckblasprozess zu einem Kunststoffbehältnis umgeformt wird.

Vorteilhaft ist die erfindungsgemäße Vorrichtung in einem Aseptikblock insbesondere in Kombination mit einer anschließend positionierten aseptischen Streckblasmaschine angeordnet.

Die Vorteile insbesondere einer Vorformlingsentkeimung bestehen darin, dass der Kunststoffvorformling im Vergleich zum gefertigten Behältnis eine geringe Oberfläche aufweist, welche sterilisiert werden muss. Auf diese Weise ist auch der Aufwand beim Aufsprühen der Farbstofflösung geringer. Weiterhin weisen die Kunststoffvorformlinge im Vergleich zum fertiggeblasenen Behältnis eine einfachere Geometrie auf und weisen insbesondere wenig oder keine Hinterschneidungen auf. Dies ist insbesondere beim Aufbringen des Farbstoffes (beispielsweise durch Sprühen)aber auch bei der Applizierung mit Strahlung von Vorteil.

Weiterhin ist die Handhabung von Kunststoffvorformlingen vergleichsweise einfach, wobei auch eine kleinere Teilung möglich ist, sodass insgesamt eine kompaktere Maschinentechnik eingesetzt werden kann.

Es wird jedoch darauf hingewiesen, dass die vorliegende Erfindung auch für andere Behältnisse und andere Kunststoffbehältnisse, wie Kunststoffflaschen eingesetzt werden kann.

Weitere Vorteile und Ausführungsformen ergeben sich aus den beigefügten Zeichnungen. Darin zeigen:
- Fig. 1: eine schematische Ansicht einer Anlage zum Herstellen von Behältnissen;
- Fig. 2: eine Detailansicht zur Veranschaulichung der zeitlichen Abläufe eines Sterilisationsvorgangs;
- Fig. 3: eine Einrichtung zum Sterilisieren von Kunststoffbehältnissen;
- Fig. 4: eine Detailansicht der in Fig. 3 gezeigten Vorrichtung;
- Fig. 5: eine weitere Ausführungsform einer Vorrichtung zum Sterilisieren von Behältnissen;
- Fig. 6a-6d: vier Ausführungsformen weiterer Vorrichtungen zum Sterilisieren von Behältnissen; und
- Fig. 7: eine weitere Ausführungsform einer erfindungsgemäßen Sterilisationseinrichtung

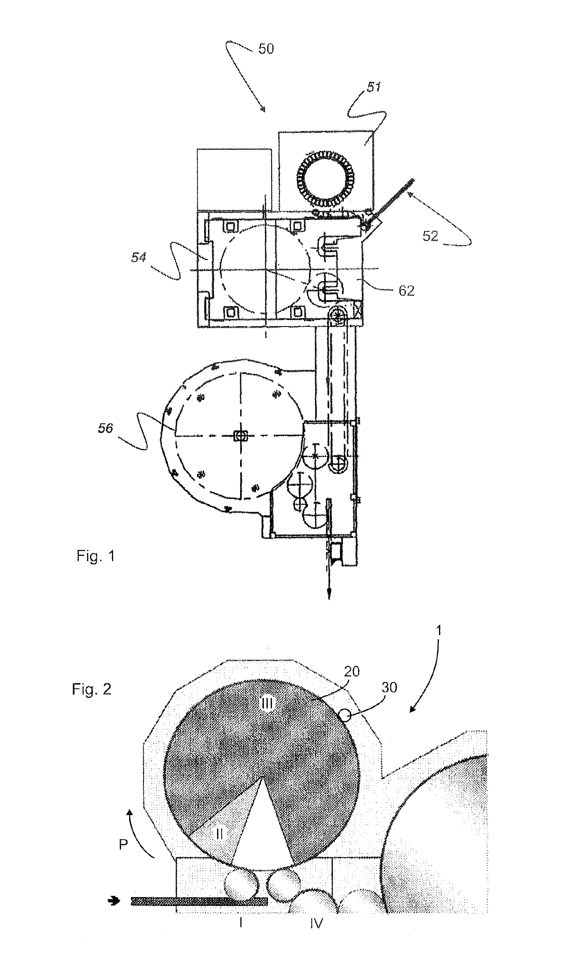

Fig. 1 zeigt eine Anlage 50 zum Behandeln von Behältern. Dabei bezieht sich das Bezugszeichen 52 auf eine Zuführeinrichtung, welche die Kunststoffvorformlinge an eine Heizeinrichtung bzw. einen Ofen 51 übergibt. Diese Heizeinrichtung 51 kann dabei ein Mikrowellenofen sein, innerhalb dessen die Behältnisse bzw. Kunststoffvorformlinge entlang eines kreisförmigen Pfades transportiert und in Resonatoren erwärmt werden. Es wäre jedoch auch denkbar, dass die Vorrichtung IR - Heizelemente, welche auch als Heizkavitäten zum vereinzelten Erwärmen der Kunststoffvorformlinge ausgebildet sein können, aufweist.

An diese Heizeinrichtung 51 schließt sich eine Streckblasmaschine 54 an, innerhalb derer die Kunststoffvorformlinge zu Kunststoffbehältnissen erwärmt werden. Anschließend werden die Behältnisse mit einer Füllmaschine 56 befüllt und abtransportiert.

Die erfindungsgemäße Vorrichtung 1 dient, wie oben erwähnt, bevorzugt zum Sterilisieren von Kunststoffvorformlingen. So wäre es möglich, dass die Kunststoffvorformlinge bereits während des Transports in der Zuführeinrichtung 52 sterilisiert werden, bevorzugt jedoch werden die Kunststoffvorformlinge während des Erwärmens in der Heizeinrichtung 51 sterilisiert. Daneben wäre es auch möglich, die Kunststoffvorformlinge nach Verlassen der Heizeinrichtung 51 und während des Transports zu der Streckblasmaschine 54 zu sterilisieren. Auch wäre eine Sterilisation der gefertigten Behältnisse nach Verlassen der Streckblasmaschine 54 möglich.

Weiterhin wäre es möglich, dass die Kunststoffvorformlinge in einem Sterilraum bzw. Reinraum 62 geführt werden und innerhalb dieses Reinraums sowohl erwärmt werden, als auch zu Kunststoffbehältnissen umgeformt werden. Die Sterilisation kann dabei, wie oben erwähnt, in diesem Sterilraum 62 erfolgen, aber auch beispielsweise unmittelbar vor Eintreten in diesen Sterilraum 62. Es wäre jedoch auch möglich, die Sterilisationseinrichtung 1 als eigenes Modul auszuführen, welches beispielsweise auch bei bestehenden Anlagen ergänzt werden kann. Weiterhin kann die Anlage 50 noch weitere Sterilisationseinheiten aufweisen, beispielsweise zum Sterilisieren der Außenoberflächen der Kunststoffvorformlinge und dergleichen.

Fig. 2 zeigt schematisch den Ablauf eines erfindungsgemäßen Sterilisationsverfahrens. Entlang eines Zuführabschnitts I können die Kunststoffvorformlinge in die Sterilisationsanlage 1 einlaufen und im Bereich des Abschnitts II mit dem fließfähigen Medium beaufschlagt und anschließend bestrahlt werden. Das Bezugszeichen P kennzeichnet dabei den Transportpfad der Kunststoffvorformlinge. In dem Abschnitt III kann das nunmehr aktivierte fließfähige Medium auf die Kunststoffvorformlinge einwirken und in dem Abschnitt IV können die behandelten Kunststoffvorformlinge wieder aus der Maschine auslaufen. Dabei wäre es, wie oben erwähnt, auch möglich, dass insbesondere in den Sektionen II und III ein Erwärmen der Kunststoffvorformlinge stattfindet. Es wäre dabei möglich, dass eine Vielzahl von (nur eine schematisch dargestellt) Sterilisationsstationen 30 an einem drehbaren Träger 20 angeordnet ist.

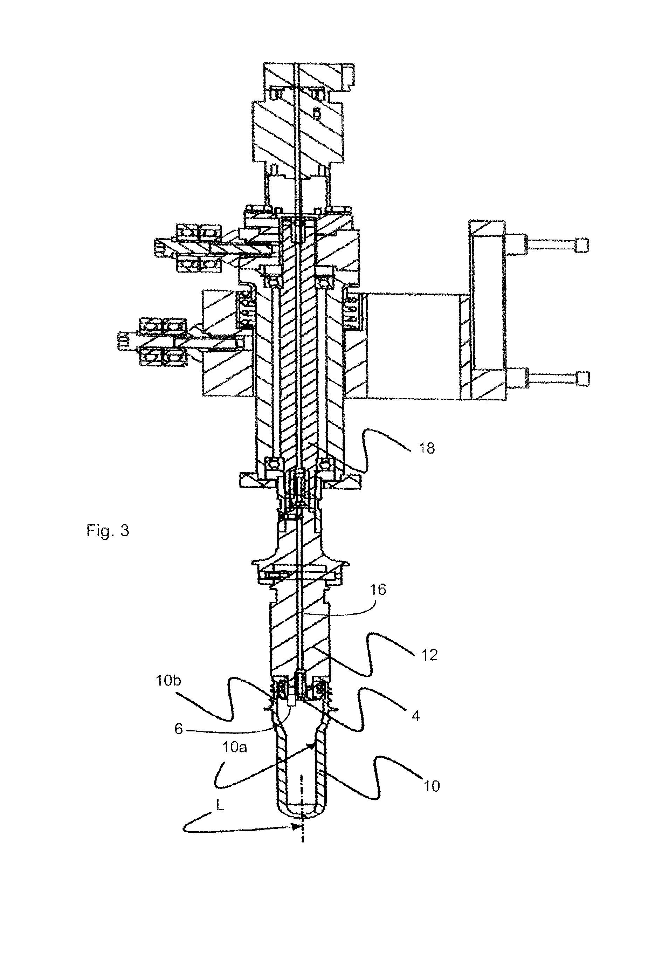

Fig. 3 zeigt eine Behandlungseinheit zum Behandeln der Kunststoffvorformlinge. Man erkennt hier einen Kunststoffvorformling 10, der an einem in seiner Gesamtheit mit 12 bezeichneten Haltedorn angeordnet ist.

Das Bezugszeichen 4 bezieht sich auf eine Beaufschlagungseinrichtung, welche das Innere des Kunststoffvorformlings 10 und insbesondere dessen Innenwandung 10a mit einem fließfähigen Mittel beaufschlagt. Das Bezugszeichen 10b kennzeichnet eine Mündung des Kunststoffvorformlings.

Weiterhin ist es möglich, dass das Haltemittel bzw. den Haltedorn 12 Bestrahlungseinrichtungen 6 aufweist, welche gezielt die Innenwandung 10a des Kunststoffvorformlings 10 mit Licht beaufschlagen, um so das fließfähige Medium zu aktivieren bzw. den Singulett - Sauerstoff zu erzeugen.

Das Bezugszeichen 16 kennzeichnet eine Zuführleitung, durch welche das fließfähige Medium zu der Beaufschlagungseinrichtung 4 transportiert werden kann. Diese Zuführeinrichtung ist hier im Inneren des Haltedorns 12 angeordnet.

Das Bezugszeichen 18 kennzeichnet eine Bewegungseinheit, die zum Bewegen der Kunststoffvorformlinge dient. Zum einen erlaubt diese Bewegungseinheit 18 eine Hub- und Senkbewegung, um den Kunststoffvorformling 10 entlang seiner Längsachse L beispielsweise von oben in den Resonator einer Erwärmungseinrichtung einzuführen und auch wieder herauszuziehen.

Weiterhin kann die Bewegungseinrichtung 18 auch dazu dienen, um den Kunststoffvorformling 10 - insbesondere während dessen Erwärmung - bezüglich seiner Längsachse L zu drehen. Die Beaufschlagungseinrichtung 4 trägt dabei das fließfähige Medium im Wesentlichen auf der gesamten Innenoberfläche des Kunststoffvorformlings auf. Nach Bestrahlung mit der Bestrahlungseinrichtung kann das fließfähige Medium seine sterilisierende Wirkung entfalten und die Innenwandung 10a des Kunststoffvorformlings sterilisieren.

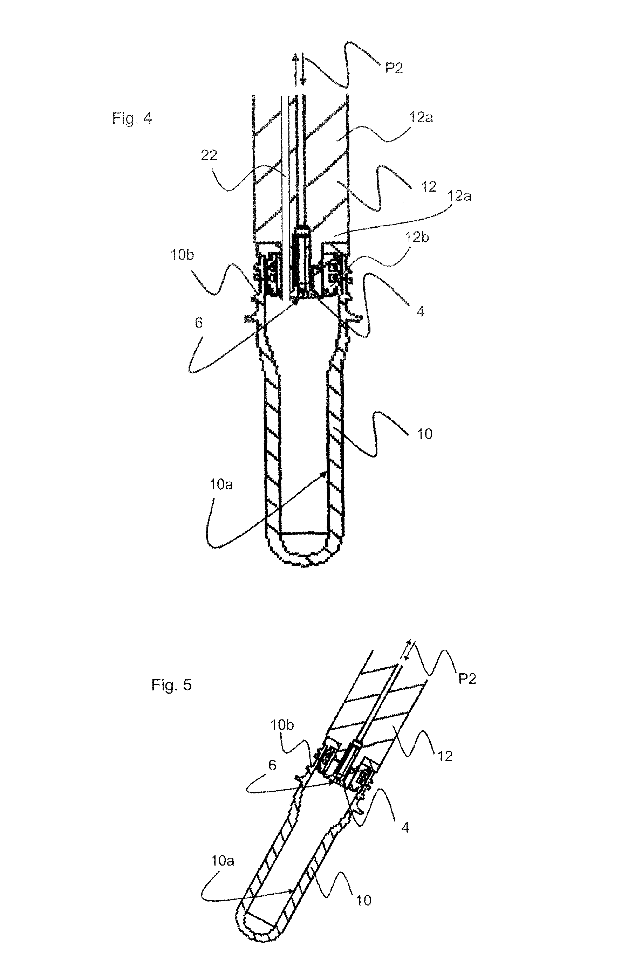

Fig. 4 zeigt eine vergrößerte Darstellung der in Fig. 3 gezeigten Vorrichtung. Auch hier erkennt man wieder das Haltemittel 12, an dem der Kunststoffvorformling 10 über sein Gewinde 10b gehalten wird. Dieser Innengreifer erzeugt durch sein das Anliegen an der Innenfläche einen "Schatten" bzw. eine Fläche, die ggfs nicht durch die Strahlung bzw. die Beaufschlagung erreicht wird.

Es sollte also darauf geachtet werden, dass diese Flächen so klein wie möglich sind bzw. es keine Schatten gibt. Alternativ kann der Vorformling oder der Behälter auch außen gehalten und transportiert werden. Auch wäre es möglich, den Innengreifer transparent auszugestalten, damit zumindest die Strahlung durch diesen hindurch tritt. Die Beaufschlagung könnte bereits erfolgen, bevor dieser (transparente) Innengreifer den Kunststoffvorformling übernimmt.

Weiterhin ist hier ein Abführkanal 22 erkennbar, über den die Abfuhr eines inbesonderen gasförmigen Mediums möglich ist. Wenn über die Beaufschlagungseinrichtung 4 das fließfähige Medium in den Kunststoffvorformling 10 eindringt, verdrängt es auf diese Weise insbesondere Luft bzw. Sterilluft innerhalb des Kunststoffvorformlings, welche dann über die Abführleitung 22 abgeführt werden kann. Es wäre weiterhin auch möglich, das Haltemittel 12 zweiteilig auszuführen, beispielsweise mit einem drehfesten Bereich 12a und einem sich diesem gegenüber drehenden Bereich 12b. Auf diese Weise wäre es möglich, den Kunststoffvorformling 10 zu drehen, während die Beaufschlagungseinrichtung 4 drehfest gehalten wird. Auf diese Weise kann durch eine Drehung des Kunststoffvorformlings auch in Umfangsrichtung die Innenwandung 10a des Kunststoffvorformlings mit dem fließfähigen Medium benetzt werden. Weiterhin wäre es auch möglich, dass die Beaufschlagungseinrichtung 4 bzw. die Düse jeweils über Zu- und oder Ableitungen mit dem Sterilisationsmedium beaufschlagt wird und/oder dieses aus dem Kunststoffvorformling 10 absaugt, um insbesondere an der Innenoberfläche 10d des Kunststoffvorformlings 10 durch zusätzliche Erwärmung seine sterilisierende Wirkung zu entwickeln.

Fig. 5 zeigt eine weitere Darstellung einer erfindungsgemäßen Vorrichtung. Man erkennt, dass hier der Kunststoffvorformling 10 schräg gestellt ist. Diese Ausführungsform ist insbesondere von Vorteil, wenn der Kunststoffvorformling entlang einer kreisförmigen Bahn bewegt wird. Einerseits kann auf diese Weise besser eine Deformation des erwärmten Kunststoffvorformlings verhindert werden, und auf der anderen Seite erlaubt diese Gestaltung auch eine günstigere Benetzung der gesamten Innenoberfläche 10a des Kunststoffvorformlings. Es wäre hierbei wiederum möglich, den Kunststoffvorformling auch bezüglich seiner Längsachse zu drehen, sodass auf diese Weise eine noch günstigere Verteilung des Sterilisationsmediums auf der Innenwandung des Kunststoffvorformlings erreicht wird.

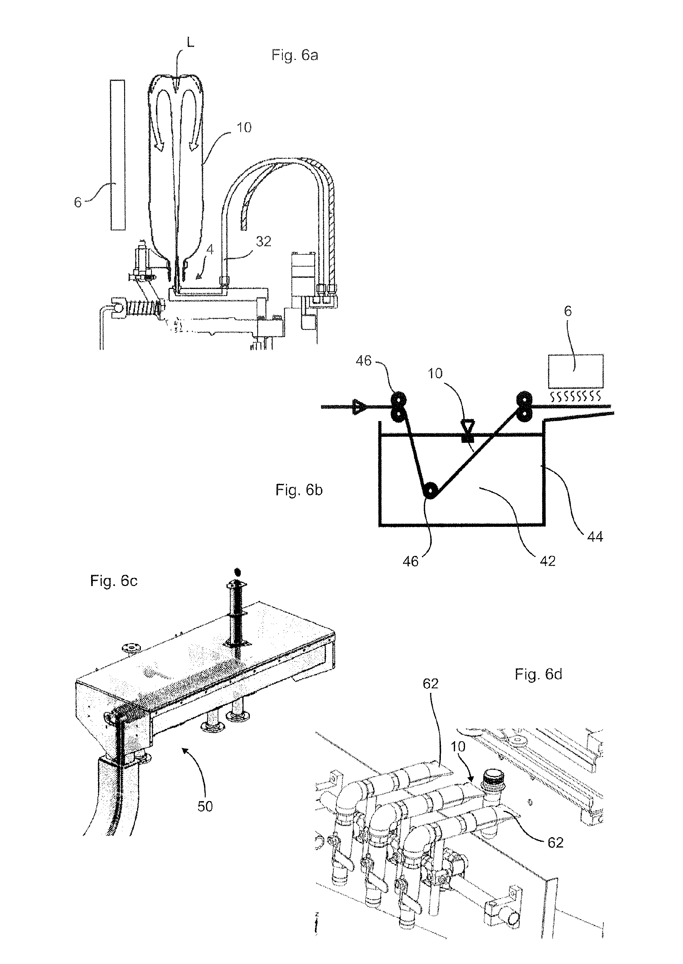

Die Fig. 6a bis 6d zeigen vier weitere mögliche Vorgehensweisen beim Sterilisieren von Verpackungsmitteln. Bei der in Fig. 6a gezeigten Variante, welche insbesondere auch zum Sterilisieren von bereits gefertigten Behältnissen 10 dient, kann auf das vorhandene Prinzip von Spülern (Rinsern) zurückgegriffen werden. Über einen Zuführungskanal 32, wobei hier auch ein Ein- oder Mehrkanalsystem vorgesehen sein kann, wird der Farbstoff (Sensibilisator) in das von dem Haltemittel 14 gehaltene Kunststoffbehältnis 10 gesprüht und verteilt. Die Verteilung kann dabei beispielsweise als Dispersion oder Lösung erfolgen.

Dabei kann das Verpackungsmittel bzw. hier Behältnis 10 auch über Kopf bzw. hängend vorliegen. Das innen und/oder außen benetzte Behältnis wird hier anschließend von der Bestrahlungseinrichtung 6 mit Licht bestimmter Wellenlänge bestrahlt und somit sterilisiert. Anschließend kann noch ein weiterer Spülvorgang erfolgen. Die Bestrahlungseinrichtung 6 kann hier vollumfänglich um das Behältnis herum angeordnet sein, es wäre jedoch auch möglich, dass sich das Behältnis 10 bezüglich seiner Längsachse L dreht.

Die in Fig. 6b gezeigte Variante ist insbesondere für folienartige Verpackungsmittel 10 geeignet. Dabei wird das Verpackungsmittel durch ein Behältnis 44, in dem sich das fließfähige Medium befindet, geführt. Zu diesem Zweck ist eine Vielzahl von Rollen 46 vorgesehen, um welche das Verpackungsmittel 10 verläuft.

Anschließend wird das Verpackungsmittel ebenfalls mit der hier stationär angeordneten Bestrahlungseinrichtung 6 bestrahlt. Diese Vorgehensweise könnte auch Anwendung finden für Granulate oder auch Verschlüsse.

Bei der in Fig. 6c gezeigten Variante ist ein Fördersystem 50 vorgesehen, welches eine Vielzahl von Schnecken und/oder Führungen in einem geschlossenen System aufweist, um die Verpackungsmittel zu fördern. Die Bestrahlungseinrichtung (nicht gezeigt) kann hier auch abgeschottet von dem eigentlichen Fördersystem 50 ausgeführt sein. Bei dem Verpackungsmittel 10 kann es sich hier insbesondere um Behältnisverschlüsse handeln. Diese können beispielsweise mit ihrer Innenseite nach oben transportiert und dabei zu nächst mit dem fließfähigen Medium beaufschlagt und anschließend mit der Bestrahlungseinrichtung bestrahlt werden.

Fig. 6d zeigt eine weitere Vorgehensweise zum Bestrahlen von Kunststoffvorformlingen. Hier ist eine Vielzahl von Düsenstöcken 62 vorgesehen, mittels denen der Kunststoffvorformling 10 mit dem fließfähigen Medium an seiner Außenwandung beaufschlagt wird. Daneben können auch hier (nicht gezeigte) Bestrahlungseinrichtungen vorgesehen sein, welche das fließfähige Medium auf der Außenwandung des Kunststoffvorformlings aktivieren.

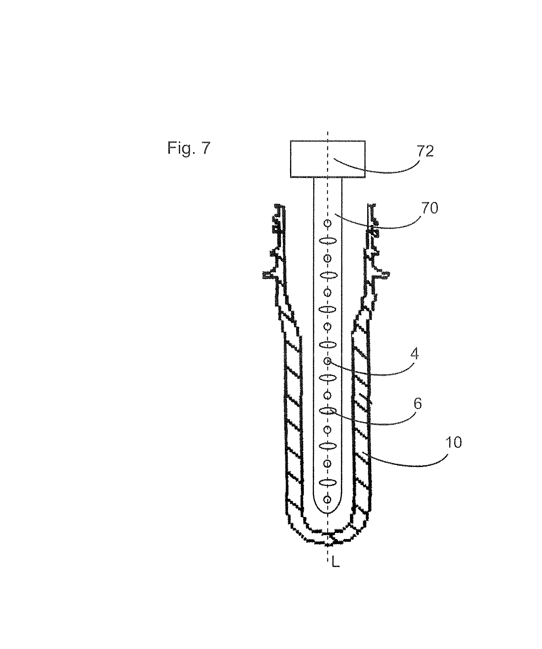

Fig. 7 zeigt eine weitere Ausführungsform einer Sterilisation von Kunststoffvorformlingen 10. Dabei ist hier ein stangenartiger Körper 70 vorgesehen, der in das Innere der Kunststoffvorformlinge 10 eingeschoben wird. Dabei wäre es möglich, dass sich der stangenartige Körper entlang seiner Längsrichtung bewegt, es wäre jedoch auch möglich, dass der Kunststoffvorformling bewegt wird.

In diesem stangenartigen Körper 70 sind hier sowohl die Beaufschlagungseinrichtungen 4 in Form von Öffnungen vorgesehen, als auch die Bestrahlungseinrichtungen 6, die beispielsweise als LEDs in diesen stangenartigen Körper 70 eingegossen werden können oder in anderer Weise in diesen integriert (durch Verschraubungen, Passsitze und dergleichen) sein können. Dabei wäre es möglich, dass die Beaufschlagungseinrichtungen 4 und die Bestrahlungseinrichtungen 6 hier jeweils voll umfänglich um den stangenartigen Körper 70 angeordnet sind, es wäre jedoch auch möglich, dass diese nur in eine Richtung weisen, und der stangenartige Körper 70 sich bezüglich der Längsrichtung L des Kunststoffvorformlings 10 dreht.

Auch wäre es möglich, dass der stangenartige Körper 70 drehfest gehalten ist und sich umgekehrt der Kunststoffvorformling dreht. Der stangenartige Körper könnte hier beispielsweise aus einem Kunststoff gefertigt sein, was insbesondere für Anwendungen in Verbindung mit mikrowellenbasierten Heizeinrichtungen von Vorteil wäre. Auch am unteren Ende des stangenartigen Körpers könnten Beaufschlagungseinrichtungen 4 und ggfs. auch Bestrahlungseinrichtungen 6 vorgesehen sein.

Daneben wäre es, insbesondere bei transparenten Kunststoffvorformlingen, auch möglich, dass die Bestrahlungseinrichtungen 4 wieder außerhalb des Kunststoffvorformlings angeordnet sind.

Das Bezugszeichen 72 kennzeichnet grob schematisch einen Antrieb für den stangenartigen Körper, der hier zum Einfahren des stangenartigen Körpers in den Kunststoffvorformlingen dient und ggfs. auch eine Drehbewegung des stangenartigen Körpers 70 bewirken kann.

Eine Benetzung der Kunststoffvorformlinge könnte auch derart erfolgen, dass diese zunächst vollständig in ein Bad mit dem fließfähigen Medium eingetaucht und so mit diesem befüllt werden und anschließend, insbesondere durch Wenden der Kunststoffvorformlinge wieder entleert werden. In diesem Fall kann das fließfähige Medium für eine vorgegebene Zeit auf die Innenwandung der Kunststoffvorformlinge einwirken und anschließend können diese (von innen oder aussen) bestrahlt werden, um Singulett - Sauerstoff zu erzeugen. Der Sauerstoff kann dabei aus der Umgebungsluft stammen, es wäre jedoch auch möglich, zusätzlich Sauerstoff hinzuzufügen. Dabei wäre es auch möglich, dass die Kunststoffvorformlinge an Bestrahlungseinrichtungen vorbeigeführt werden und sich bevorzugt dabei auch um ihre Längsachse drehen.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Vorrichtung
- 4: Beaufschlagungseinrichtung
- 6: Bestrahlungseinrichtungen
- 10: Kunststoffvorformling
- 10a: Innenwandung
- 10b: Mündung des Kunststoffvorformlings, Gewinde
- 12: Haltedorn
- 12a: drehfester Bereich
- 12b: drehender Bereich
- 14: Haltemittel
- 16: Zuführleitung
- 18: Bewegungseinheit
- 20: drehbarer Träger
- 22: Abführleitung
- 30: Sterilisationsstation
- 32: Zuführungskanal
- 44: Behältnis
- 46: Rollen
- 50: Anlage, Fördersystem
- 51: Ofen, Heizeinrichtung
- 52: Zuführeinrichtung
- 54: Streckblasmaschine
- 56: Füllmaschine
- 62: Reinraum, Sterilraum, Düsenstock
- 70: stangenartiger Körper
- 72: Antrieb
- L: Längsachse
- P: Transportpfad
- I, II, III, IV: Abschnitt, Bereich

## Patentansprüche

1. Verfahren zum Sterilisieren von Verpackungsmitteln (10) und insbesondere von Kunststoffvorformlingen (10), wobei die Verpackungsmittel (10) mit einer Transporteinrichtung (2) transportiert werden und wobei wenigstens ein Bereich des Verpackungsmittels (10) mit einer Beaufschlagungseinrichtung (4) mit einem fließfähigen Medium beaufschlagt wird,
**dadurch gekennzeichnet, dass**
das auf dem Bereich aufgebrachte Medium einer Bestrahlung mit elektromagnetischer Strahlung ausgesetzt wird, um Singulett - Sauerstoff zu erzeugen wobei dieser Singulett - Sauerstoff zum Sterilisieren des Verpackungsmittels (10) dient.

2. Verfahren nach Anspruch 1,
**dadurch gekennzeichnet, dass**
das Verpackungsmittel während des Transports mit dem fließfähigen Medium beaufschlagt wird und/oder der Singulett - Sauerstoff während des Transports des Verpackungsmittels erzeugt wird.

3. Verfahren nach wenigstens einem der vorangegangenen Ansprüche,
**dadurch gekennzeichnet, dass**
das fließfähige Medium einen Farbstoff enthält.

4. Verfahren nach wenigstens einem der vorangegangenen Ansprüche,
**dadurch gekennzeichnet, dass**
die Beaufschlagung des Verpackungsmittels (10) vor und/oder während und/oder unmittelbar nach einer Erwärmung des Verpackungsmittels (10) erfolgt.

5. Verfahren nach wenigstens einem der vorangegangenen Ansprüche,
**dadurch gekennzeichnet, dass**
das fließfähige Medium dem Verpackungsmittel (10) über eine Zuführleitung (16) und/oder wenigstens eine Düse zugeführt wird.

6. Verfahren nach wenigstens einem der vorangegangenen Ansprüche,
**dadurch gekennzeichnet, dass**
das fließfähige Medium eine positiv oder eine negativ geladene Flüssigkeit ist.

7. Verfahren nach wenigstens einem der vorangegangenen Ansprüche,
**dadurch gekennzeichnet, dass**
die Beaufschlagung und Sterilisation der Verpackungsmittel in einem kontinuierlichen Prozess, insbesondere auf einer Rundläufermaschine erfolgt.

8. Vorrichtung (1) zum Sterilisieren von Verpackungsmitteln (10) und insbesondere Kunststoff - Vorformlingen (10), mit einer Transporteinrichtung (2), welche das Verpackungsmittel (10) entlang eines vorgegebenen Transportpfades transportiert, mit einer Beaufschlagungseinrichtung (4), welche wenigstens einen Bereich des Verpackungsmittels mit einem fließfähigen Medium beaufschlagt,
**dadurch gekennzeichnet, dass**
die Vorrichtung (1) eine Bestrahlungseinrichtung (6) aufweist, welche den mit dem fließfähigen Medium beaufschlagten Bereich des Verpackungsmittels (10) einer elektromagnetischen Strahlung zur Erzeugung von Singulett - Sauerstoff aussetzt.

9. Vorrichtung (1) nach Anspruch 8,
**dadurch gekennzeichnet, dass**
die Transporteinrichtung ein Haltemittel (12) zum Halten des Verpackungsmittels (10) aufweist und in dieses Haltemittel (12) bevorzugt auch die Beaufschlagungseinrichtung (4) und/oder die Bestrahlungseinrichtung (6) integriert ist.

10. Vorrichtung (1) nach Anspruch 9,
**dadurch gekennzeichnet, dass**
das Haltemittel (12) in eine Mündung des Verpackungsmittels (10) eingreift.

11. Vorrichtung (1) nach wenigstens einem der vorangegangenen Ansprüche,
**dadurch gekennzeichnet, dass**
die Vorrichtung eine Erwärmungseinrichtung (51) zum Erwärmen des Verpackungsmittels (10) aufweist.
